# EUROPEAN PATENT SPECIFICATION

(11) **EP 1 227 765 B1**
(45) Date of publication and mention of the grant of the patent: **04.05.2005**
(21) Application number: 99952216.2
(22) Date of filing: 11.11.1999
(51) Int. Cl.: A61B 17/72

(54) **RADIALLY EXPANDABLE INTRAMEDULLARY NAIL**
RADIAL AUFWEITBARER MARKNAGEL
CLOU INTRAMEDULLAIRE A EXPANSION RADIALE

(43) Date of publication of application: 07.08.2002
(73) Proprietor: SYNTHES AG Chur, 7002 Chur (CH)
(72) Inventor: PERREN, Stephan, CH-7260 Davos (CH); HEHLI, Markus, CH-7276 Frauenkirch (CH)
(74) Representative: Lusuardi, Werther Giovanni, Dr.
(86) International application number: PCT/CH1999/000532
(87) International publication number: WO 2001/034045

(56) References cited:
- WO-A-97/18769
- DE-A- 2 542 263
- FR-A- 2 633 345
- GB-A- 2 268 068
- US-A- 4 790 304

## Description

This invention concerns an intramedullary nail in accordance with the precharacterising portion of Claim 1.

Various types of intramedullary nails are already known in the state of the art, which are expandable in a limited section of the nail in order to allow the fixation of the nail against the bone cortex, e.g. by means of radially deploying a number of blades in the distal portion of the intramedullary nail.

Such an intramedullary nail is known from FR 2 633 345 DUCHARME. This known intramedullary nail is provided with two radially expandable sections that are attached to a nut each at their rear ends while their front ends may expand when the straining screw is tightende.

The disadvantages of these known devices consist in:
a) The fact that the nail is not extrafocal and so is not consistent with the state of art concept of fracture treatment. The largest expansion of the nail is in the fracture area, which affects negatively the endosteal vascularisation just in the fracture area, i.e. the most important area;
b) The largest expansion of the nail in the fracture area generates forces which have the tendency to separate the fragments in comminuted fractures or, in the case of longitudinal non-dislocated fractures to increase the gap and to dislocate fragments which leads to loosening of the fixation.

A further intramedullary nail is known from DE 25 42 263 LASSEN. This known intramedullary nail is provided with two radially expandable sections, one at its proximal end an the other one at its distal end. The two radially expandable sections are each realised through a plurality of slots extending parallel to the longitudinal axis from the proximal and distal end. The expansion of the two radially sections is effected by means of a conical nut each being axially displacable through a straining screw. The drawback of this typ of intramedullary nail are its limited expandable sections.

The invention as claimed aims at solving the above described problems and disadvantages.

The present invention provides an intramedullary nail as defined in claim 1.

The nail according to the invention allows to obtain the following advantages:
a) The expandable parts of the nail are located extrafocally, leaving the fracture area minimally affected from radial forces;
b) the nail is particularly strong in the middle part - between the proximal and distal sections - of the nail
c) no fluoroscopy is needed for distal nor proximal locking since the expandable parts are fastened via tightening a nut on a thread in the longitudinal direction of the intramedullary nail.

The intramedullary nail according to the invention comprises a first radially expandable section provided in the proximal section adjacent to the head of the intramedullary nail, a second radially expandable section provided in the distal section adjacent to the tip of the intramedullary nail and a non-expandable middle section provided between said two radially expandable sections of the intramedullary nail.

The head provides a distraction mechanism, by means of which said two radially expandable sections are distractible transverse to said longitudinal axis.

In a preferred embodiment of the intramedullary nail according to the invention the non-expandable section of the intramedullary nail has a length in the range of 10 to 25 mm, preferably in the range of 15 to 22 mm.

Furthermore, the intramedullary nail consists of a solid nail core with a head and a tip. In the proximal section adjacent to the head a thread is provided. The middle section of the core is configured with a smaller diameter than the end section of the core such that the middle section and the end section are separated by an annular abutment. Apart from the core the intramedullary nail according to this embodiment comprises two longitudinally slotted tubular pieces that provide the first and second radially expandable sections, two unslotted tubular pieces and a nut. These elements are slid over the core in the following sequence:
- a slotted tubular piece
- an unslotted tubular piece
- a slotted tubular piece
- an unslotted tubular piece
such that adjacent to the tip of the nail a first slotted tubular piece is provided. The second slotted tubular piece is separated from the first one by means of an unslotted tubular piece. Adjacent to the end of the nail a nut with an interior thread corresponding to a thread on the core is mounted such allowing to compress the tubular pieces along the longitudinal axis. When these element are aligned on the core a screwing of the nut onto the thread on the core - by means of a driving means such as a spanner, wrench or similar tool engaged to suitable means at the nut such as two parallel sides, hexagonal sides, a hexagon socket or the like - and holding up the core against rotation - by means of a second tool inserted into corresponding suitable means at the core such as a slot, hexagon socket or the like - will produce an axial force or compression on the tubular pieces as soon as the first slotted tubular piece abuts against the abutment of the core and the nut is tightened. Under that compression force the belts between the cuts at the slotted tubular pieces begin to buckle and deform radially such causing a radial expansion of the slotted tubular pieces. By this effect the nail expands at a first radially expandable section in the proximal section adjacent to the head of the nail and in a second radially expandable section in the distal section adjacent to the tip of the nail.

The intramedullary nail according to the invention differentiates from the above embodiment therein that it provides instead of the core a hollow cylindrical or prismatical sleeve extending along the longitudinal axis and surrounding a rodlike locking element coaxially to the longitudinal axis. The locking element comprises a shaft with a thread in the proximal section towards the head of the nail and a projection forming an abutment in the distal section. In the distal section the sleeve provides a slot penetrating the side wall through to the bore such that the projection of the locking element may slide within said slot in the direction of the longitudinal axis. Once the tubular elements are aligned on the sleeve as mentioned above the tightening of the nut on the thread on the locking element while holding up the locking element against rotation causes a compression force onto the tubular elements clamped between the nut and the abutment producing the same effect as described in the above embodiment of the intramedullary nail.

When the nut is tightened a sliding of the proximal section and distal section on the first and second transverse abutments respectively is produced. If the intramedullary channel of the femur has a larger in diameter than the intramedullary nail the proximal section and distal section will expand transversely to the longitudinal axis until firm fixation of the bone fragments is reached. This radial expansion is possible because the first and second transverse abutments enclose an angle of between 5° and 85° with the longitudinal axis such that upon applying a force in the axial direction by means of tightening the nut the proximal section, the middle section and the distal section slide on the abutments providing a sliding component orthogonal to the longitudinal axis what causes a radial expansion of the intramedullary nail.

The middle section may provide sharp radial teeth that prevent axial displacement of the bone fragments after fixation of the nail.

The various features of novelty which characterize the invention are pointed out with particularity in the claims annexed to and forming part of this disclosure. For the better understanding of the invention, its operating advantages and specific objects attained by its use, reference should be had to the accompanying drawings, examples and descriptive matter in which are illustrated and described preferred embodiments of the invention.

In the drawings:
Fig. 1 shows a schematic representation of an intramedullary nail in the non-expanded state;
Fig. 2 shows an exploded view of the intramedullary nail according to Fig. 1;
Fig. 3 shows a schematic representation of one of the expandable sections of the intramedullary nail according to Fig. 1 in the non-expanded and in the expanded state;
Fig. 4 shows a schematic representation of an intramedullary nail in the expanded state;
Fig. 5 shows an intramedullary nail according to the invention;
Fig. 6 shows a variation of the nut to be used with the intramedullary nail according to Fig. 5.

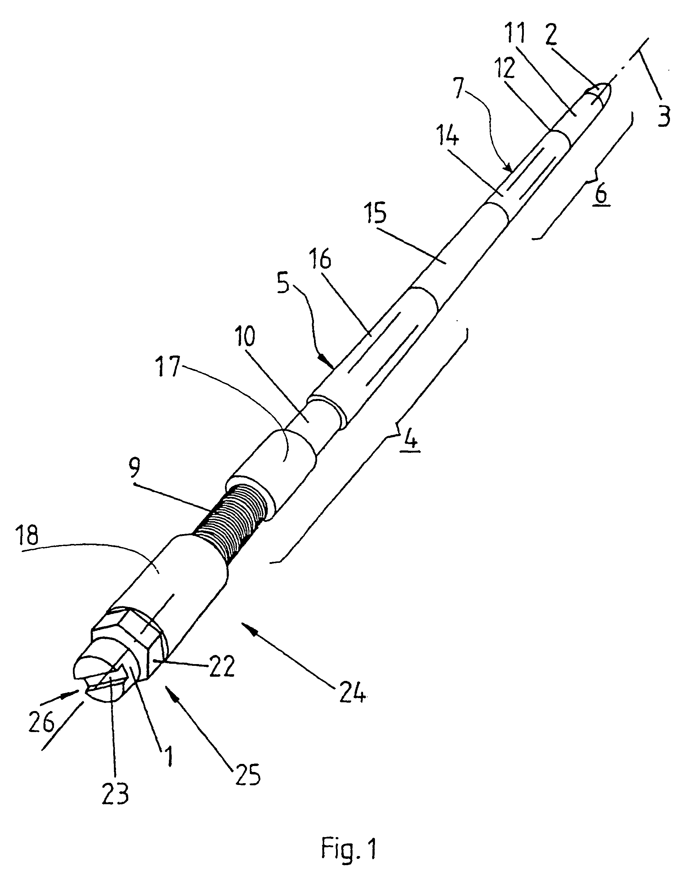

Figures 1 to 4 show a first embodiment of an intramedullary nail which consists of a solid nail core 10 with a head 1 and a tip 2 and a longitudinal axis 3. In the proximal section 4 adjacent to the head 1 a tread 9 is provided. The middle section 13 of the core 10 has smooth surface and a tapering diameter towards the distal section 6 adjacent to the tip 2. The middle section 13 of the core 10 - with a minor diameter - and the end section 11 of the core 10 - with a larger diameter - are separated by an annular abutment 12.

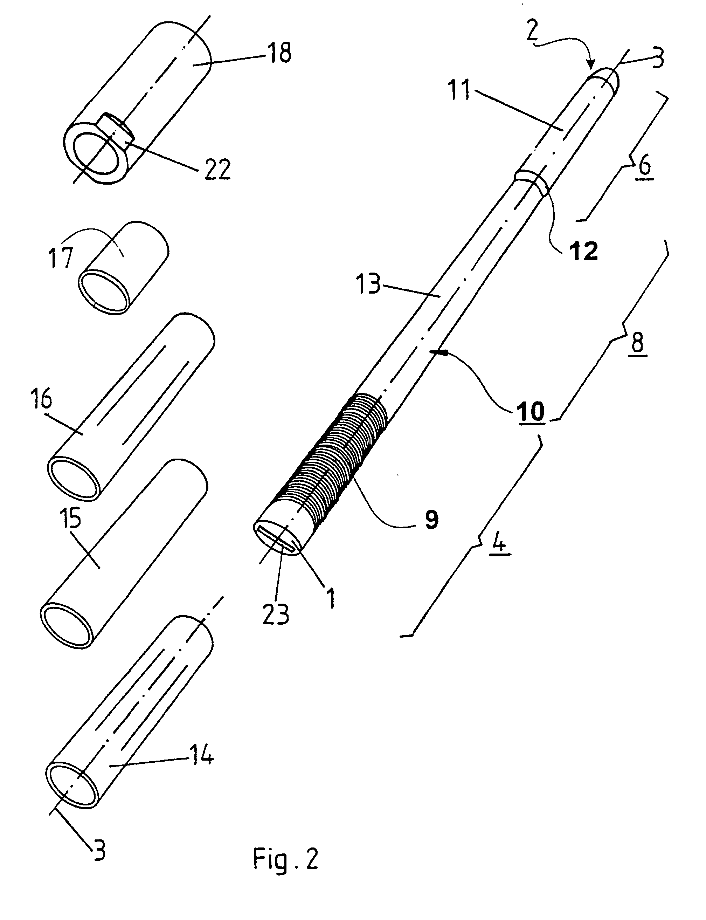

As shown in Fig. 2 the following elements are slid over the core 10 in the following sequence:
- a slotted tubular piece 14
- an unslotted tubular piece 15
- a slotted tubular piece 16
- an unslotted tubular piece 17
- a nut 18 with an inner thread corresponding with thread 9.

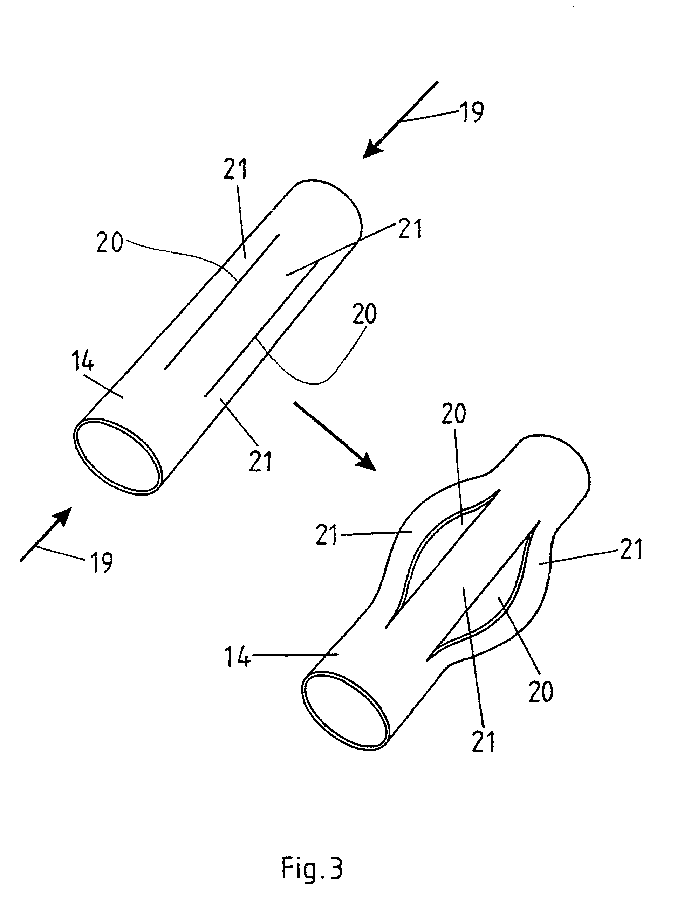

When these element are aligned on the core 10 a screwing of the nut 18 - by means of the two parallel sides or hexagonal sides 22 - on the thread 9 and holding up the core 10 - by means of the slot 23 - will produce an axial force or compression (as indicated by arrows 19 in Fig. 3) on the tubular pieces (17,16,15,14) as soon as the slotted tubular piece 14 abuts against the abutment 12 of the core 10. As shown in Fig. 3 the cuts 20 of the slotted tubular piece 14 - as well as those of slotted tubular piece 16 - will cause an expansion of the slotted tubular piece 14 by radially deforming the belts 21 located between the cuts 20.

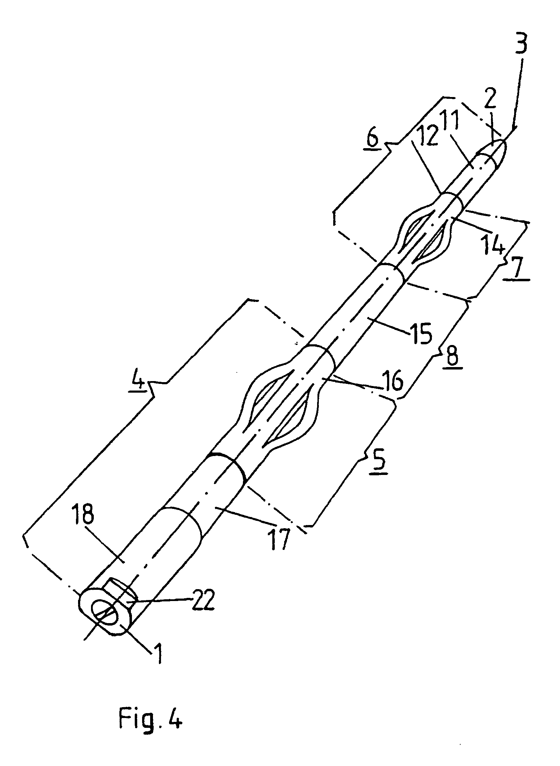

By this effect the nail expands - as shown in Fig. 4 - at a first radially expandable section 5 in the proximal section 4 adjacent to the head 1 of the nail and in a second radially expandable section 7 in the distal section 6 adjacent to the tip 2 of the nail. Instead of the two parallel sides or hexagonal sides 22 and the slot 23 for tightening the nut 18 two hexagon sockets may be placed.

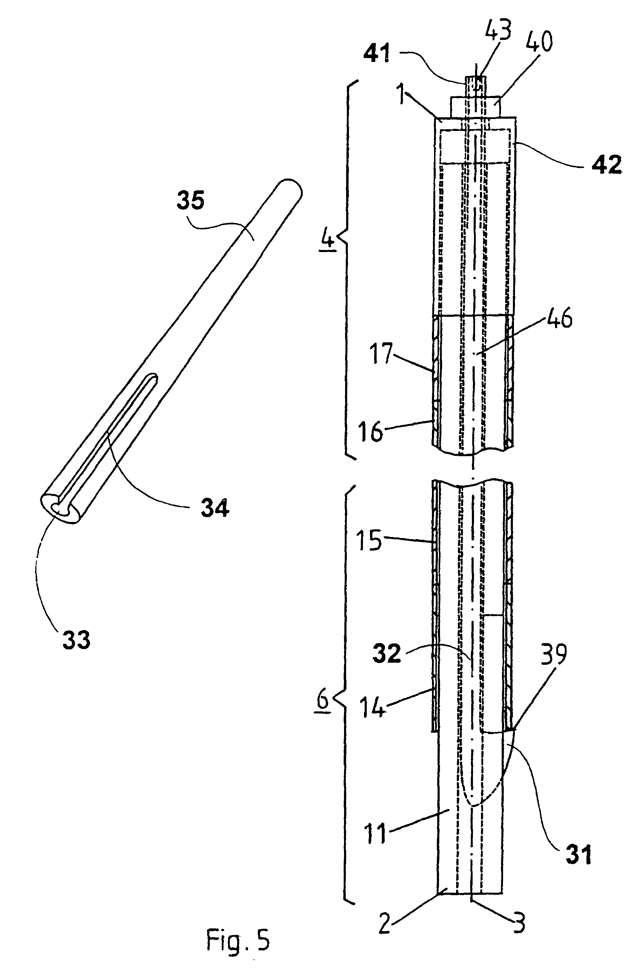

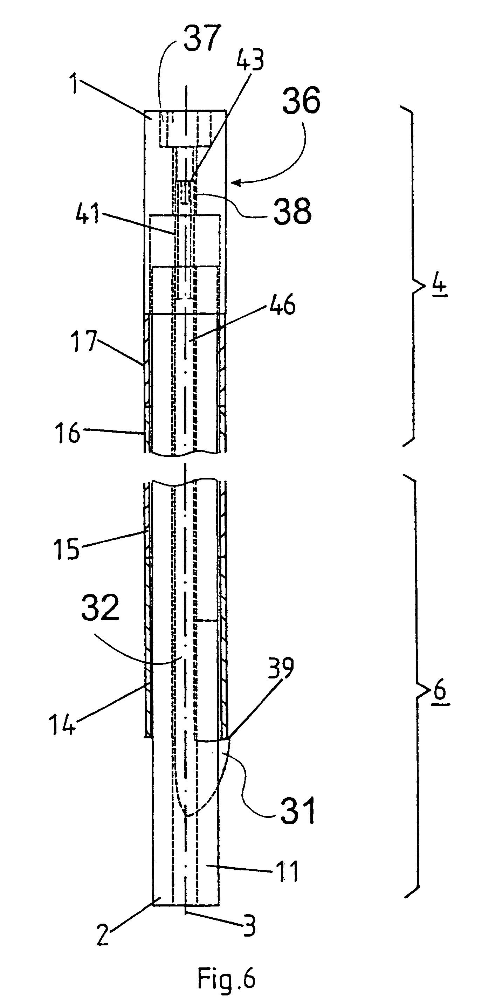

Fig. 5 and 6 show an embodiment of the intramedullary nail which differentiates from the embodiment shown in Fig. 1-4 therein that it provides instead of the core 10 a hollow cylindrical sleeve 35 extending along the longitudinal axis 3 and surrounding a locking element 32 coaxially to the longitudinal axis 3. The locking element 32 comprises a shaft with a thread 41 in the proximal section 4 and in the distal section 6 a projection 31 forming an abutment 39. In the distal section 6 the sleeve 35 provides a slot 34 penetrating the side wall through to the bore 33 such that the projection 31 of the locking element 32 may slide within said slot 34 in the direction of the longitudinal axis 3. The following elements are slid over the sleeve 35 in the following sequence:
- a slotted tubular piece 14
- an unslotted tubular piece 15
- a slotted tubular piece 16
- an unslotted tubular piece 17
- an end cap 42
- a nut 40 with an inner thread corresponding with thread 41.

Once these elements are aligned on the sleeve 35 the tightening of the nut 40 on the thread 41 while counter holding the locking element 32 by means of the hexagon socket 43 axially compresses the tubular pieces 14;15;16;17 as soon as the slotted tubular piece 14 abuts against the abutment 39 formed by the projection 31 of the locking element 32. Equally as in case of the first embodiment the belts 21 of the slotted tubular pieces (14;16) buckle and radially expand under the axial forces 19 (Fig. 3). Instead of the nut 40 and the end cap 42 another end cap 36 providing in interior thread 38 corresponding with thread 41 and a hexagon socket 37 may be used (Fig. 6).

While the foregoing description and drawings represent the preferred embodiments of the present invention, it will be obvious for those skilled in the art that various changes and modifications may be made therein without departing from the scope of the present invention.

## Claims

1. Intramedullary nail for fixation of bone fractures with a head (1), a tip (2) and a longitudinal axis (3), comprising
A) a first radially expandable section (5) is provided in the proximal section (4) adjacent to the head (1) of the intramedullary nail;
B) a second radially expandable section (7) is provided in the distal section (6) adjacent to the tip (2) of the intramedullary nail;
C) a non-expandable middle section (8) is provided between said two radially expandable sections (5,7) of the intramedullary nail, whereby
D) the radially expandable sections (5;7) are realised by slotted tubular pieces (14;16); and
E) the non-expandable middle section (8) is realised by a unslotted tubular piece (15),
**characterised in that**
F) the intramedullary nail comprises a hollow cylindrical sleeve (35) extending along the longitudinal axis (3); and
G) a locking element (32) being surrounded by the sleeve (35); whereby
H) the locking element (32) comprises a shaft with a thread (41) in the proximal section (4) and a projection (31) forming an abutment (39) in the distal section (6); and
I) the distal section (6) of the sleeve (35) is provided with a slot (34) penetrating the side wall through to the bore (33) such that the projection (31) of the locking element (32) may slide within said slot (34) in the direction of the longitudinal axis (3).

2. Intramedullary nail according to claim 1, **characterized in that** the head (1) is provided with a distraction mechanism (24), by means of which said two radially expandable sections (5,7) are distractible transverse to said longitudinal axis (3).

3. Intramedullary nail according to claim 1 or 2, **characterized in that** said non-expandable section (8) of the intramedullary nail has a length in the range of 10 to 25 mm.

4. Intramedullary nail according to claim 3, **characterized in that** said non-expandable section (8) of the intramedullary nail has a length in the range of 15 to 22 mm.

5. Intramedullary nail according to one of the claims 1 to 4, **characterized in that** the slotted tubular pieces (14;16) are configured slidable on the sleeve (35) and prevented from sliding on the end section (11) towards the tip (2) by means of the abutment (39).

6. Intramedullary nail according to one of the claims 2 to 5, **characterized in that** the distraction mechanism (24) comprises a thread (41) on the locking element (32) adjacent to the head (1) and a nut (40) with an interior thread corresponding with thread (41).

7. Intramedullary nail according to claim 6, **characterized in that** the nut (40) comprises means (25) for engagement of a driving means.

8. Intramedullary nail according to claim 6 or 7, **characterized in that** the locking element (32) comprises means (43) for engagement of a holding means.

9. Intramedullary nail according to one of the claims 2 to 8, **characterized in that** the distraction mechanism (24) comprises a thread (41) on the locking element (32) adjacent to the head (1) and an end cap (36) with an interior thread (38) corresponding with thread (41).

10. Intramedullary nail according to claim 9, **characterized in that** the end cap (36) comprises means (37) for engagement of a driving means.

11. Intramedullary nail according to claim 9 or 10, **characterized in that** the locking element (32) comprises means (43) for engagement of a holding means.

## Patentansprüche

1. Marknagel zur Fixierung von Knochenfrakturen mit einem Kopf (1), einer Spitze (2) und einer Längsachse (3), welcher folgendes umfasst:
A) Ein erster, radial spreizbarer Abschnitt (5) ist in dem proximalen Abschnitt (4) angrenzend an den Kopf (1) des Marknagels vorgesehen;
B) ein zweiter, radial spreizbarer Abschnitt (7) ist in dem distalen Abschnitt (6) angrenzend an die Spitze (2) des Marknagels vorgesehen;
C) ein nichtspreizbarer Mittelabschnitt (8) ist zwischen den beiden radial spreizbaren Abschnitten (5;7) des Marknagels vorgesehen, wobei
D) die radial spreizbaren Abschnitte (5;7) durch geschlitzte, rohrförmige Stücke (14; 16) gebildet werden; und
E) der nichtspreizbare Mittelabschnitt (8) durch ein ungeschlitztes, rohrförmiges Stück (15) gebildet wird, **dadurch gekennzeichnet, dass**
F) der Marknagel eine sich entlang der Längsachse (3) erstreckende, hohlzylindrische Hülse (35); und
G) ein von der Hülse (35) umgebenes Verriegelungselement (32) umfasst; wobei
H) das Verriegelungselement (32) einen Schaft mit einen in dessen proximalem Abschnitt (4) ausgebildeten Gewinde (41), sowie einen in dem distalen Abschnitt (6) einen Stoss (39) bildenden Vorsprung (31) umfasst; und I) der distale Abschnitt (6) der Hülse (35) mit einem in deren Seitenwand ausgebildeten Schlitz (34) versehen ist, welcher sich bis hin zu der Bohrung (33) erstreckt, so dass der Vorsprung (31) des Verriegelungselements (32) in dem Schlitz (34) in der Richtung der Längsachse (3) verschiebbar ist.

2. Marknagel nach Anspruch 1, **dadurch gekennzeichnet, dass** der Kopf (1) mit einem Distraktionsmechanismus (24) versehen ist, durch welchen die beiden radial spreizbaren Abschnitte (5,7) in Querrichtung zu der Längsachse (3) distrahierbar sind.

3. Marknagel nach Anspruch 1 oder 2, **dadurch gekennzeichnet, dass** der nichtspreizbare Abschnitt (8) des Marknagels eine Länge in einem Bereich von 10 bis 25 mm aufweist.

4. Marknagel nach Anspruch 3, **dadurch gekennzeichnet, dass** der nichtspreizbare Abschnitt (8) des Marknagels eine Länge in einem Bereich von 15 bis 22 mm aufweist.

5. Marknagel nach einem der Ansprüche 1 bis 4, **dadurch gekennzeichnet, dass** die geschlitzten, rohrförmigen Stücke (14;16) entsprechend ausgelegt sind, um auf der Hülse (35) verschiebbar zu sein, und durch den Stoss (39) daran gehindert werden, auf dem Endabschnitt (11) in Richtung zu der Spitze (2) verschoben zu werden.

6. Marknagel nach einem der Ansprüche 2 bis 5, **dadurch gekennzeichnet, dass** der Distraktionsmechanismus (24) ein auf dem Verriegelungselement (32) angrenzend an den Kopf (1) ausgebildetes Gewinde (41) und eine Mutter (40) mit einem dem Gewinde (41) entsprechenden Innengewinde umfasst.

7. Marknagel nach Anspruch 6, **dadurch gekennzeichnet, dass** die Mutter (40) Mittel (25) zur Aufnahme eines Antriebsmittels umfasst.

8. Marknagel nach Anspruch 6 oder 7, **dadurch gekennzeichnet, dass** das Verriegelungselement (32) Mittel (43) zur Aufnahme eines Haltemittels umfasst.

9. Marknagel nach einem der Ansprüche 2 bis 8, **dadurch gekennzeichnet, dass** der Distraktionsmechanismus (24) ein auf dem Verriegelungselement (32) angrenzend an den Kopf (1) ausgebildetes Gewinde (41) und eine Abschlusskappe (36) mit einem dem Gewinde (41) entsprechenden Innengewinde (38) umfasst.

10. Marknagel nach Anspruch 9, **dadurch gekennzeichnet, dass** die Abschlusskappe (36) Mittel (37) zur Aufnahme eines Antriebsmittels umfasst.

11. Marknagel nach Anspruch 9 oder 10, **dadurch gekennzeichnet, dass** das Verriegelungselement (32) Mittel (43) zur Aufnahme eines Haltemittels umfasst.

## Revendications

1. Clou intramédullaire permettant de fixer des fractures osseuses, comportant une tête (1), une pointe (2) et un axe longitudinal (3), ledit clou comprenant :
A) une première partie radialement expansible (5) est prévue dans la partie proximale (4) contiguë à la tête (1) du clou intramédullaire;
B) une deuxième partie radialement expansible (7) est prévue dans la partie distale (6) contiguë à la pointe (2) du clou intramédullaire;
C) une partie centrale non-expansible (8) est prévue entre les deux parties radialement expansibles (5;7) du clou intramédullaire,
D) les parties radialement expansibles (5;7) étant formées par des pièces tubulaires (14;16) pourvues de fentes; et
E) la partie non-expansible (8) étant formée par une pièce tubulaire (15) dépourvue de fentes, **caractérisé en ce que**
F) le clou intramédullaire comprend une douille (35) en forme de cylindre creux s'étendant le long de l'axe longitudinal (3); et
G) un élément de verrouillage (32) entouré par la douille (35);
H) l'élément de verrouillage (32) comprenant un corps avec un filet (41) formé sur sa partie proximale (4) ainsi qu'une saillie (31) formant, sur sa partie distale (6), un about (39); et I)la partie distale (6) de la douille (35) étant pourvue d'une fente (34) ménagée dans la paroi latérale de celle-ci et s'étendant jusqu'au trou (33), de sorte que la saillie (31) de l'élément de verrouillage (32) peut coulisser dans la fente (34) dans la direction de l'axe longitudinal (3).

2. Clou intramédullaire selon la revendication 1, **caractérisé en ce que** la tète (1) est pourvue d'un mécanisme d'écartement (24), lequel permet d'écarter les deux parties radialement expansibles (5;7) dans le sens transversal à l'axe longitudinal (3).

3. Clou intramédullaire selon la revendication 1 ou 2, **caractérisé en ce que** la partie non-expansible (8) du clou intramédullaire présente une longueur comprise entre 10 et 25 mm.

4. Clou intramédullaire selon la revendication 3, **caractérisé en ce que** la partie non-expansible (8) du clou intramédullaire présente une longueur comprise entre 15 et 22 mm.

5. Clou intramédullaire selon l'une des revendications 1 à 4, **caractérisé en ce que** les pièces tubulaires (14;16) pourvues de fentes sont conçues de manière à pouvoir coulisser sur la douille (35) et à être empêchées par l'about (39) de coulisser sur l'extrémité (11) en direction de la pointe (2).

6. Clou intramédullaire selon l'une des revendications 2 à 5, **caractérisé en ce que** le mécanisme d'écartement (24) comprend un filet (41) formé sur l'élément de verrouillage (32) de manière contiguë à la tête (1) et un écrou (40) pourvu d'un filet intérieur correspondant au filet (41).

7. Clou intramédullaire selon la revendication 6, **caractérisé en ce que** l'écrou (40) comprend des moyens (25) permettant de recevoir un moyen d'entraînement.

8. Clou intramédullaire selon la revendication 6 ou 7, **caractérisé en ce que** l'élément de verrouillage (32) comprend des moyens (43) permettant de recevoir un moyen de fixation.

9. Clou intramédullaire selon l'une des revendications 2 à 8, **caractérisé en ce que** le mécanisme d'écartement (24) comprend un filet (41) formé sur l'élément de verrouillage (32) de manière contiguë à la tête (1) et un chapeau de fermeture (36) pourvu d'un filet intérieur (38) correspondant au filet (41).

10. Clou intramédullaire selon la revendication 9, **caractérisé en ce que** le chapeau de fermeture (36) comprend des moyens (37) permettant de recevoir un moyen d'entraînement.

11. Clou intramédullaire selon la revendication 9 ou 10, **caractérisé en ce que** l'élément de verrouillage (32) comprend des moyens (43) permettant de recevoir un moyen de fixation.
